# EUROPEAN PATENT APPLICATION

(11) **EP 1 548 104 A1**
(43) Date of publication of application: **29.06.2005**
(21) Application number: 03792846.2
(22) Date of filing: 26.08.2003
(51) Int. Cl.: C12N 15/00, C12M 1/00, C07H 21/00, G01N 33/50

(54) **NUCLEIC ACID RECOVERY CHIP AND NUCLEIC ACID RECOVERY DEVICE**

(30) Priority: 26.08.2002 JP 2002245905
(71) Applicant: Japan Science and Technology Agency, Kawaguchi-shi, Saitama 332-0012 (JP)
(72) Inventor: YASUDA, Kenji, Koto-ku, Tokyo 135-0052 (JP); ICHIKI, Takanori, Tsurugashima-shi, Saitama 350-2203 (JP); OKANO, Kazunori, Shiki-shi, Saitama 353-0004 (JP)
(74) Representative: Calamita, Roberto
(86) International application number: PCT/JP2003/010765
(87) International publication number: WO 2004/018665

(57) **Abstract**

On a glass plate being transparent at specified wavelengths there are provided a laminated region being absorptive at the specified wavelengths; means for applying voltage to the region, the region exhibiting electrical conductivity; means for binding nucleic acids onto the region; a container for accommodating cells on the region; means for culturing cells in the container; means for observing the cells; and means for effecting localized dissociation and recovery of nucleic acid components bound on the region by heat, the heat generated locally only in the vicinity of focused light by irradiating the region with focused light of the specified wavelengths. For clarifying the distribution of nucleic acid components in cells of specified condition or the distribution of nucleic acid components in each cell of a tissue cell mass there is provided means for selectively separating and recovering nucleic acid components of specified range in each cell of specified cellular condition.

## Description

### Technical Field

The present invention relates to a nucleic acid recovery chip obtained by observing cells while culturing, and selectively separating and recovering nucleic acid components in a specified region of the cells when the cells get to have a specific state, and a nucleic acid recovery device.

### Background Art

Recently, development of efficient and highly sophisticated methods and means for separating and analyzing DNAs, nucleic acids, and further proteins as biological specimens have proceeded rapidly.

For example, the known DNA chip is a method for detecting a specific base sequence utilizing the micro patterning technique and the DNA complementation. This is for analyzing the sequence of the DNA in the specimen by patterning oligonucleotides having various sequences as an array on a substrate and detecting the bonding position of the DNA specimen on the substrate. Moreover, the PCR reaction is well known as a technique comprising the repetition of three reactions of separation of the duplex DNA structure (95°C, 30 seconds), annealing with an oligonucleotide (37°C, 20 seconds), and the complemental strand synthesis by a DNA polymerase (72°C, 2 minutes), for amplifying a minute amount of DNA pieces to several hundred thousand times. On the other hand, as a technique for generating the temperature rise in a local area of a fluid, there is a technique of using a light beam. A technique for cutting a protein sub unit associated body by generating the local temperature rise in a µm order minute area in the vicinity of the fine particles by laser trapping of fine particles having the light absorption is reported by Washizu, et al. in page 111 to page 114 of the Institute of Electrostatics lecture articles (1994). Moreover, the present inventors have already proposed in the Japanese Patent Application No. 10-163214 an apparatus and a method for locally separating and recovering the complementary strand DNAs by fixing different nucleic acid probes in a plurality of areas on a substrate, bonding complementary strand DNAs in the specimen DNAs, and using a heat of a convergent light beam.

On the other hand, there is no prior art of culture controlling the solution environment of culture cells and the physical contact among the cells. Then, the present inventors have invented a technique for newly selecting only one specific cell and culturing the cell as a cell line so as to solve the problems, a technique for controlling the solution environment condition of the cells in the case of observing cells, a technique for controlling the cell concentration in the container constantly, and a technique for observing the culture while specifying the cells interacting with each other, and have filed the same as the Japanese Patent Application No. 2000-356827.

However, according to the conventional gene analysis technique according to the DNA chips, capture and observation by complementally bonding primers of about 20 bases formed on a substrate and the single strand DNA/RNA in the specimen have been regarded important so that the technique for analyzing the mRNA intracellular distribution in a specific cellular state (specific period in a cell cycle) and the intracellular nucleic acid components per cell unit has not been considered in the present situation. Then, the same is applied to the various kinds of the conventional methods (apparatus) for separating and analyzing biology related specimens.

In order to analyze the structure of a living creatures, its activity or the like, the separation and the analysis of the nucleic acid components in a specific area in a specific cellular state as mentioned above are essential, however, the means therefor has barely been discussed.

Then, an object of the present invention is to provide a novel technical means for selectively separating and recovering nucleic acid components in a specific range of each cell in a specific cellular state in order to reveal the nucleic acid component distribution in a cell in a specific state or the nucleic acid component distribution in a specific range in each cell in a specific cellular state.

### Disclosure of the Invention

In order to solve the above-mentioned problems, the present invention provides firstly a nucleic acid recovery chip in a cell, having an electrically conductive area disposed on a transparent substrate with a nucleic acid binding part and a cell accommodating container part provided on the area, comprising a means for applying an electric potential to the above-mentioned electrically conductive area, and a means for culturing a cell in the cell accommodating container part, characterized in that the above-mentioned electrically conductive area has the absorption of a light beam of a specific wavelength such that it generates a heat locally by the light irradiation of the specific wavelength so as to locally dissociate and recover the nucleic acid components bound on the electrically conductive area.

Moreover, the present invention provides secondly a nucleic acid recovery chip characterized in comprising an electrically conductive area and an upper electrically conductive part disposed facing thereto as the means for applying the electric potential to the electrically conductive area, thirdly a nucleic acid recovery chip characterized in comprising a housing container part for enveloping the cell accommodating container part for allowing passage of a cell culturing solution as the means for culturing the cell in the cell accommodating container part, and fourthly the nucleic acid recovery chip according to any of claims 1 to 3, characterized in that at least one cell accommodating container part for accommodating a cell is provided.

Then, the present invention provides fifthly a nucleic acid recovery device comprising any of the above-mentioned nucleic acid recovery chips, characterized in comprising an optical system for directing a light beam of a specific wavelength to the electrically conductive area of the nucleic acid recovery chip for locally generating a heat, and a power source system for applying the electric potential to the area, sixthly a nucleic acid recovery device characterized in comprising an observation system for observing the cellular state, and seventhly a nucleic acid recovery device characterized in comprising a transportation system for a culturing solution of a cell.

### Brief Description of Drawings

FIG. 1 is a schematic diagram of an embodiment showing an example of the basic configuration of a nucleic acid recovery chip according to the present invention.
FIG. 2 is a schematic diagram showing an example of the nucleic acid recovery procedure in the cells, using a nucleic acid recovery chip according to the present invention.
FIG. 3 is a schematic diagram showing an embodiment as an example of an optical system and a solution sending system using the nucleic acid recovery chip according to the present invention.

The numerals in the figures denote the following.
- 100: nucleic acid recovery chip
- 101: optically transparent substrate
- 102: thin film having the optical absorption
- 103: optically transparent substance wall not toxic to the cells or the like
- 104: specimen such as a cell
- 105: cellular container
- 106: optically transparent container
- 107, 108: pipe
- 109: conductive layer
- 110: tunnel
- 111: power source module
- 112, 107, 305: objective lens
- 113: nucleic acid probe
- 201: substrate surface
- 202: nucleic acid probe
- 203: cell
- 204: protein
- 205: nucleic acid probe
- 206, 209: nucleic acid
- 208: locally heated area
- 301: light source
- 302, 209, 312: filter
- 303: condenser lens
- 304: stage with the temperature adjusting function
- 306: movable dichroic mirror
- 308: light source
- 310: dichroic mirror
- 311: mirror
- 313: camera
- 315: motor for moving the stage
- 316: solution vessel
- 317: supply device
- 318: distributing device with the PCR reaction function
- 319: capillary electrophoresis device
- 320: waste solution reservoir

### Best Mode for Carrying Out the Invention

The present invention has the characteristics as mentioned above. Hereinafter, embodiments thereof will be explained.

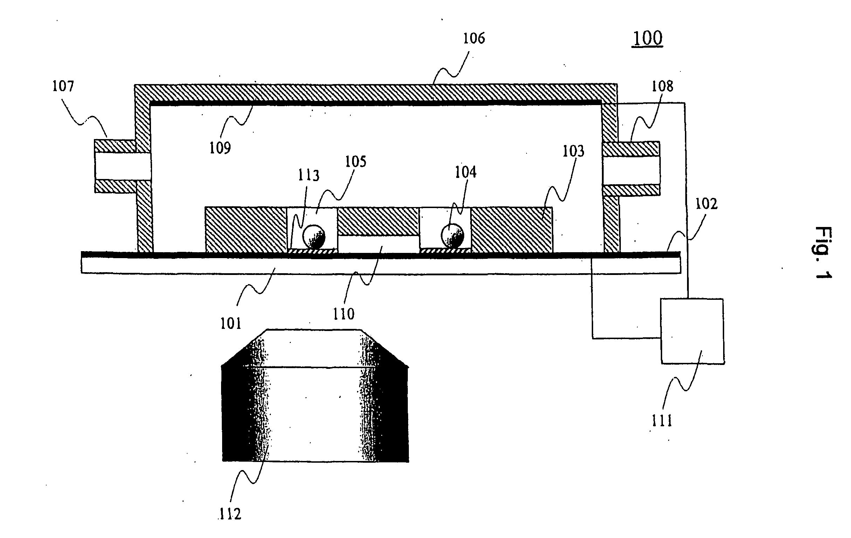

First, an example of the basic configuration of a nucleic acid recovery chip according to the present invention will be explained with reference to an embodiment of FIG. 1. As a nucleic acid recovery chip 100, a thin film layer 102 having the optical absorption, made of a chromium deposition layer or the like is disposed on an optically transparent substrate 101 such as a slide glass. In the case of the observation with a transmitted light beam, it is preferable that the film thickness of the thin film layer 102 is about thin enough so as not to completely absorb the light beam without irregularity. For example, in the case of a chromium, depositing by a 50 Å film thickness, the transmitted light beam in the visible range is about 70 % so that it can be used for the application of the present invention without any problem. Additionally, any metal selected from the group consisting of Ti, V, Fe, Co, Ni, Mo and W may be used as well. According to these metals, by the covalent bond of an oxide layer formed on the metal layer surface with a silanol group, a nucleic acid probe layer 113 can be fixed on the substrate surface. A container wall 103 for culturing a cell 104 is laminated on the light absorbing thin film layer 102. The size and the arrangement of the container can be selected optionally according to the kind of the cell. For example, in the case of a nerve cell, about a 50 µm or more width is preferable. Moreover, although the cell cannot move between the containers, a path 110 may be provided so that an axon or the like can be bonded with another cell. Moreover, the height of the container wall 103 should be sufficiently high so as to prevent the cell from moving over. For example, in the case of a nerve cell, the height may be 20 µm or more. Especially in the case of using an agarose as the material for the container wall 103, it has no bonding property with respect to the cell nor it is not a signal substance to the cell so that not only it is harmless to the cell but also it poses little influence to the culturing experiment data, and thus it is optimum. In the case the culturing solution circulation is required at the time of culturing the cell 104, the solution can be introduced from a pipe 107 and the waste solution can be collected from a pipe 108 with an optically transparent container 106 having a structure for totally covering the cell culturing area including the container wall 103 placed thereon. At the time, an electrically conductive area 109 is added on the upper surface in the container 106 so as to be paired with an area 102 to provide an electrode. As to the thickness of the area 109, it is deposited thinly to the extent to have the optical transmission property like the area 102. Here, the cellular state in the container 105 can be observed continuously, using an objective lens 112. Moreover, by directing a convergent light beam of a near infrared beam to the substrate surface via the objective lens, a heat can be generated locally so that the nucleic acid components bound with the above-mentioned nucleic acid probe layer 113 can be thermally modified selectively so as to be recovered. By using a power source module 111, an electric field can be applied between the area 102 and the area 109. Moreover, according to this embodiment, although the area 102 is provided as an electrode, a plurality of electrode array to be operated independently for each cell container may be used as well.

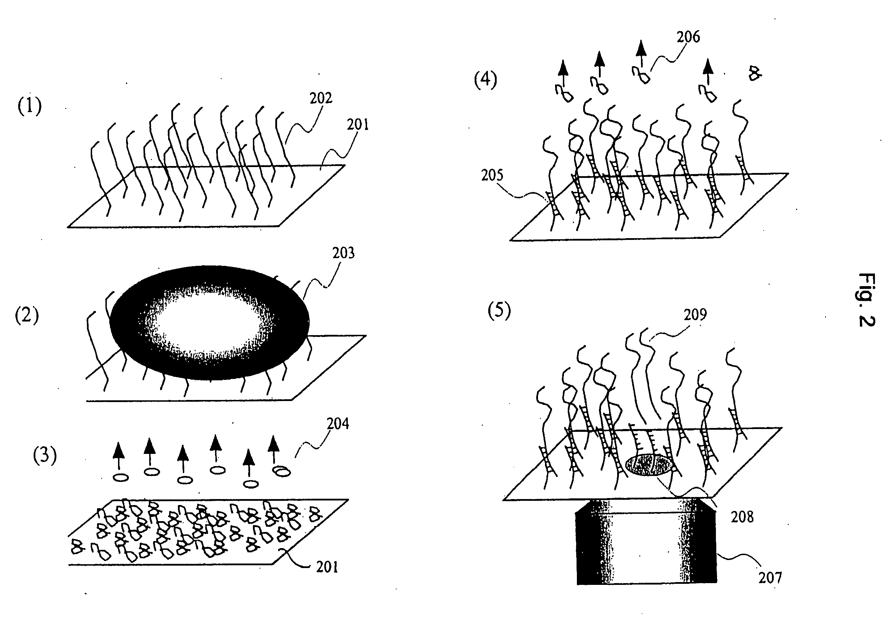

FIG. 2 is a schematic diagram showing an example of a nucleic acid recovery procedure in a cell using a nucleic acid recovery chip according to the present invention. FIG. 2(1) is a schematic diagram showing the substrate surface state before the introduction of a cell. A nucleic acid probe 202 is fixed on the substrate surface 201 for capturing the nucleic acid components. Here, as the nucleic acid probe 202, a sequence mainly with a poly-T for selectively recovering the mRNA may be provided, a sequence complementary to a specific nucleic acid component may be provided in the case the specific nucleic acid component is desired to be recovered, or a plurality of nucleic acid probes having different sequences may be mixed and arranged. FIG. 2(2) is a schematic diagram showing the state with a cell 203 disposed on the above-mentioned substrate surface 201. While observing the state of the cell 203, at the time it attains a specific cell state, the cell may be crushed by an infrared ray convergent light beam directed through the microscope objective lens, or in the case the electrodes of each cell culturing container are independent with each other, the cell may be crushed by applying an electric field of a direct current or an alternative current to the electrode. Or in the case cells of the all containers are crushed at the same time, the cells may be crushed by introducing a chemical such as a surfactant. FIG. 2(3) is a schematic diagram showing the separation procedure of the intracellular protein and the nucleic acid components after crushing the cell. By applying the direct current electric field between the substrate surface 201 and the counter electrode, the protein components and the nucleic acid components are separated. Since the isoelectric points differ largely for the protein and the nucleic acid components, with only the nucleic acid components attracted to the substrate surface 201, the protein components 204 can be liberated from the substrate surface so as to be eliminated by supplying the solution. Moreover, the nucleic acid components in the cell can be fixed in a form protected two dimensionally on the substrate surface 201. FIG. 2(4) shows that the nucleic acid components 206 not coupled with the nucleic acid probe 205 on the substrate surface can be eliminated thereafter by applying the direct current electric field opposite to the prior one. FIG. 2(5) is a schematic diagram showing the next procedure for locally dissociating and recovering the intracellular nucleic acid components coupled with the nucleic acid probe by heating with the convergent light beam. Since only the nucleic acid specimen 209 coupled with the nucleic acid probe in the area 208 heated by the convergent light beam directed by the objective lens 207 is dissociated selectively in the solution, by collecting the solution including the dissociated nucleic acid components in the area, the nucleic acid components in a specific area in the cell can be collected selectively.

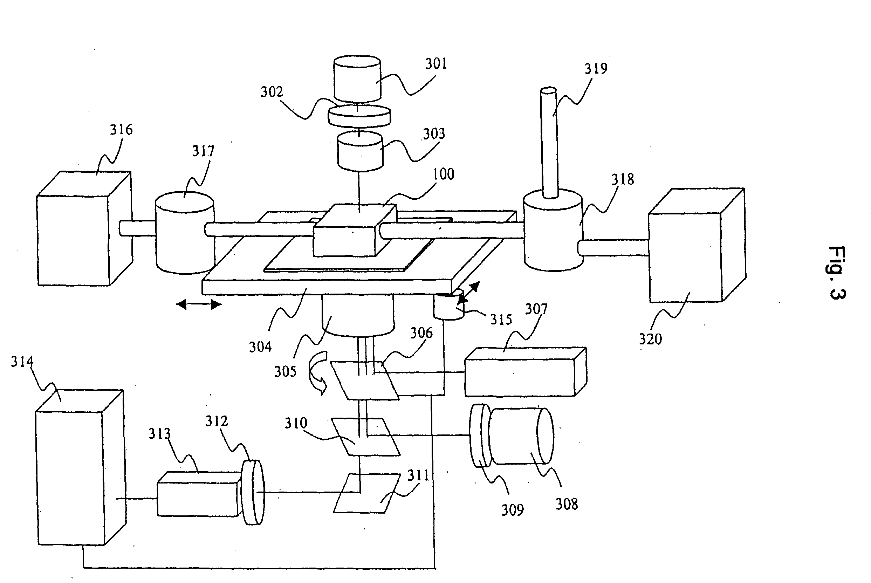

FIG. 3 schematically exemplifies the device configuration as an embodiment of an optical system and a solution sending system using the nucleic acid recovery chip according to the present invention. According to the device, in order to observe the state change while culturing the specimen such as a cell in the nucleic acid recovery chip 100, a microscope observation system, a culturing solution circulation system, and at the same time a convergent light irradiation system for selectively collecting the nucleic acid on the nucleic acid recovery chip 100 are provided. As it is shown also in FIG. 3, the nucleic acid recovery chip 100 is disposed on the optical path of the microscope observation optical system, with the nucleic acid recovery chip 100 interlocked with a culturing solution supplying and abandoning part for supplying the solution. First, the microscope observation optical system has the following configuration. A light beam outputted from the light source 301 is adjusted to have a specific wavelength by the filter 302 and it is converged by the condenser lens 303 so as to be directed to the nucleic acid recovery chip 100. The directed light beam is used as the transmitted light beam for the observation with the objective lens 305. The transmitted light image inside the nucleic acid recovery chip 100 is guided to the camera 313 after passing through the filter 312 by the mirror 311 so as to be focused on the light receiving surface of the camera. Therefore, the material of the nucleic acid recovery chip 100 is preferably a material optically transparent with respect to a light beam of the wavelength selected by the filter 302. Specifically, a glass such as a borosilicate glass and a quartz glass, a resin or a plastic such as a polystyrene, a solid substrate such as a silicon substrate, or a polymer such as an agarose are used. Moreover, particularly in the case a silicon substrate is used, a light beam of a 900 nm or more wavelength is used for the observation. Moreover, as it has been described for the light absorbing layer 102 of FIG. 1, it is preferable to use selectively a film thickness to have less than 80 % light absorption for the above-mentioned specific wavelength or a wavelength without having the absorption.

Moreover, a light beam outputted from the light source 308 is also guided to the objective lens 305 by the dichroic mirror 310 after the wavelength selection by the filter 309 so as to be used as the exciting light beam for the fluorescence observation inside the nucleic acid recovery chip 100. The fluorescence emitted from the nucleic acid recovery chip 100 is collected again by the objective lens 305 so that only the fluorescence and the transmitted light beam after cutting the exciting light beam by the filter 312 can be observed by the camera 313. At the time, by adjusting the combination of the filters 302, 309, 312, observation of only the transmitted light beam by the camera 313, observation of only the fluorescence or simultaneous observation of the transmitted light beam image and the fluorescence image can be enabled. On the optical path there is provided a mechanism for guiding the laser beam generated by the laser light source 307 to the objective lens 305 by the movable dichroic mirror 306. The laser beam is processed to be a convergent light beam by the objective lens 305 so that the nucleic acid recovery chip 100 can be heated locally. In the case of moving the light focusing point, by moving the movable dichroic mirror, the laser beam converging position inside the nucleic acid recovery chip 100 can be moved. As to the laser wavelength, a wavelength without having the water absorption or the photochemical function is preferable. For example, in the case of 1,064 nm of a Nd:YAG laser, there is no remarkable absorption with respect to water, glass, agarose or the like so that the laser beam is absorbed only in the chromium thin film layer selectively, and thus heat is generated only in the vicinity of the light focusing point of the chromium thin film layer with the light beam absorbed.

The image data obtained by the camera are analyzed by the image processing analysis device 314 so as to drive the stage moving motor 315 for moving in the X-Y direction freely for the control of the position of the movable dichroic mirror 306 and the movable XY stage with the temperature adjusting function 304 with the nucleic acid recovery chip 100 placed thereon. Thereby, the cell shape can be recognized, the cell can be pursued after the recognition so as to be placed always in the center of the image, or the focus of the image can be provided on a specific cell by adjusting the distance with respect to the objective lens. Or the movable dichroic mirror 306 or the stage with the temperature adjusting function 304 with the culturing micro chamber 100 placed thereon can be controlled by a constant time cycle, or the stage moving motor 315 can be driven by a constant interval.

The culturing solution supplying and abandoning part will be explained. It has a structure wherein the culturing solution supplied a plurality of kinds of culturing solutions of different concentrations, a reagent for crushing the cell or the like to the nucleic acid recovery chip 100 from the solution vessel 316 by the supply device 317 according to the cell state, has the liquid temperature adjusted by the temperature adjusting mechanism in the supplying device, and furthermore, the dissolved gas component adjusted by the dissolved air exchanging mechanism so that the reagent such as the culturing solution can be supplied to the nucleic acid recovery chip 100 while adjusting the flow rate. The culturing solution of the container 100 can be introduced to the capillary electrophoresis device 319 after amplifying the PCR reaction again by the distributing device with the PCR reaction function for examining the sequence of the specimen nucleic acid supplied from the nucleic acid recovery chip 100 or the waste solution can be sent to the waste solution reservoir 320.

Of course it is needless to say that the present invention is not limited to the above-mentioned embodiments and various embodiments can be employed for the details thereof.

### Industrial Applicability

As heretofore explained in detail, according to the present invention, the space distribution of the nucleic acid components in a specific state of the culturing cells can be analyzed.

## Claims

1. A nucleic acid recovery chip in a cell, having an electrically conductive area disposed on a transparent substrate with a nucleic acid binding part and a cell accommodating container part provided on the area, comprising a means for applying an electric potential to the above-mentioned electrically conductive area, and a means for culturing a cell in the cell accommodating container part, **characterized in that** the above-mentioned electrically conductive area has the absorption of a light beam of a specific wavelength such that it generates a heat locally by the light irradiation of the specific wavelength so as to locally dissociate and recover the nucleic acid components bound on the electrically conductive area.

2. The nucleic acid recovery chip according to claim 1, **characterized in** comprising an electrically conductive area and an upper electrically conductive part disposed facing thereto as the means for applying the electric potential to the electrically conductive area.

3. The nucleic acid recovery chip according to claim 1 or 2, **characterized in** comprising a housing container part for enveloping the cell accommodating container part for allowing passage of a cell culturing solution as the means for culturing the cell in the cell accommodating container part.

4. The nucleic acid recovery chip according to any of claims 1 to 3, **characterized in that** at least one cell accommodating container part for accommodating a cell is provided.

5. A nucleic acid recovery device comprising the nucleic acid recovery chip according to any of claims 1 to 4, **characterized in** comprising an optical system for directing a light beam of a specific wavelength to the electrically conductive area of the nucleic acid recovery chip for locally generating a heat, and a power source system for applying the electric potential to the area.

6. The nucleic acid recovery device according to claim 5, **characterized in** comprising an observation system for observing the cellular state.

7. The nucleic acid recovery device according to claim 5 or 6, **characterized in** comprising a transportation system for a culturing solution for a cell.
